# EUROPEAN PATENT APPLICATION

(11) **EP 2 298 937 A1**
(43) Date of publication of application: **23.03.2011**
(21) Application number: 10008553.9
(22) Date of filing: 23.05.2007
(51) Int. Cl.: C12Q 1/68

(54) **Methods and kits for linking polymorphic sequences to expanded repeat mutations**

(30) Priority: 24.05.2006 US 439858
(62) Divisional of application: 07777234.1
(71) Applicant: Medtronic, Inc., 710 Medtronic Parkway NE Minneapolis, MN 55342-5604 (US)
(72) Inventor: Van Bilsen, Paul, 6224 HW Maastricht (NL); Kaemmerer, William F., Edina, MN 55435 (US); Burright, Eric, Eagan, MN 55123 (US)
(74) Representative: Schmidt, Karsten

(57) **Abstract**

Methods and kits are provided for determining which single nucleotide polymorphism ("SNP") variant of an allele of a heterozygous patient is on the same allele as a disease-causing mutation that is at a remote region of the gene's mRNA comprising a) an allele specific reverse transcription reaction using an allele specific primer which recognizes one SNP variant, wherein further the 3' end of the primer is positioned at the SNP nucleaotide position, and b) analysis of the resulting cDNA product from the reverse transcription reaction at the region of the mutation to determine the presence or absence of the mutation on this allele specific cDNA product, wherein the allele specific primer is shorter than about 20 nucleotides.

## Description

### CROSS REFERENCE TO RELATED APPLICATION(S)

This application claims the benefit of U.S. Patent Application No. 11/439,858, filed on May 24, 2006, the entire teachings of which are incorporated herein by reference.

### FIELD OF THE INVENTION

The present invention relates generally to compositions and methods for diagnosing diseases which have an allele-specific therapy and a disease-causing mutation that is sufficiently distant from the molecular site of the therapy to require a diagnostic linking method.

### BACKGROUND OF THE INVENTION

Expansions of CAG trinucleotide repeats (CAG repeats) in coding regions of human genes cause numerous disorders by generating proteins with elongated polyglutamine (polyQ) stretches. This group of disorders includes by way of example Dystophia myotonica, Spinocerebellar ataxia type 1, Spinocerebellar ataxia type 2, Spinocerebellar ataxia type 3, Spinocerebellar ataxia type 6, Spinocerebellar ataxia type 7, Spinocerebellar ataxia type 8, Spinocerebellar ataxia type 17, Huntington disease-like 2, Spinal and bulbar muscular atrophy, Huntington disease, Dentatorubral-pallidoluysian atrophy, Oculopharyngeal dystrophy, Congenital central hypoventilation syndrome, Infantile spasms, Synpolydactyly, Cleidocranial dysplasia, Holoprosencephaly, Hand-foot-genital syndrome, Type II blephorophimosis, ptosis, and epicanthus inversus syndrome. (Wanker E.E. (2000) Biol. Chem., 381:937-942; Gusella J.F. and MacDonald,M.E. (2000) Nature Rev. Neurosci., 1:109 -115; and Usdin K. and Grabczyk,E. (2000) Cell. Mol. Life Sci., 57:914-931).

For purposes of illustration only Huntington's disease (HD) will be discussed herein. HD is a chronic neurodegenerative disorder which is inherited as an autosomal dominant trait and is characterized by chorea, dementia and personality disorder. Martin,J.B. and Gusella,J.F. (1986) N. Engl. J. Med. 315:1267-1276. The gene responsible for HD contains an expanded and unstable CAG trinucleotide repeat. Huntington's Disease Collaborative Research Group (1993) Cell 72:971-983.

The HD gene (*IT15* gene), which encodes huntingtin, a 350 kDa protein of unknown function, is located on the human chromosome 4 and consists of 67 exons. The disease-causing mutation is a CAG repeat expansion located within exon 1 of the HD gene (HD exon1). The CAG repeat is translated into a polyQ stretch. The disease manifests itself when the polyQ stretch exceeds the critical length of 37 glutamines (pathological threshold), whereas 8-35 glutamine residues in huntingtin are tolerated by neuronal cells. Experimental evidence has been presented that huntingtin fragments with polyQ tracts in the pathological range (more than 37 glutamines), but not in the normal range (20-32 glutamines), form high molecular weight protein aggregates with a fibrillar morphology *in vitro* and in cell culture model systems (Scherzinger et al. (1999) Proc. Natl Acad. Sci. USA, 96:4604-4609; and Waelter et al., (2001) Mol. Biol. Cell, 12:1393-1407). In addition, inclusions with aggregated N-terminally truncated huntingtin protein were detected in HD transgenic mice carrying a CAG repeat expansion of 115-156 units and in HD patient brains (Davies et al., (1997) Cell, 90:537-548; and DiFiglia et al., (1997) Science, 277:1990-1993), suggesting that the process of aggregate formation may be important for the progression of HD. The mechanisms, however, by which the elongated polyQ sequences in huntingtin cause dysfunction and neurodegeneration are not yet understood (Scherzinger et al., (1999); Tobin A.J. and Signer, E.R. (2000) Trends Cell Biol., 10:531-536; and Perutz M.F. (1999) Glutamine repeats and neurodegenerative diseases: molecular aspects. Trends Biochem. Sci., 24:58-63).

Unaffected individuals have repeat numbers of up to 30, while individuals at a high risk of developing HD carry more than 37 CAG repeats. Individuals with 30-37 repeats have a high risk of passing on repeats in the affected size range to their offspring (Andrew et al., (1997) Hum. Mol. Genet., 6:2005-2010; Duyao et al., (1993) Nature Genet., 4:387-392; and Snell et al., (1993) Nature Genet., 4:393-397).

It is known that patients are able to survive and live healthy lives with only one functioning copy of the *IT15* gene. Thus, selective inactivation of the allele with a disease-causing mutation should diminish or even eliminate the disease while improving the possibilities of survival in heterozygous patients.

The combination of emotional, cognitive and motor symptoms in HD contributes to an unusually high cost of care. People with Huntington's Disease require care from health professionals of many stripes, including general practitioners, neurologists, social workers, home health aides, psychologists, physical therapists, and speech/language pathologists.

Currently, there are a few diagnostic approaches for nucleic acid sequence identification. U.S. Patent Application Publication No. 20040048301 describes allele-specific primer extension in the presence of labeled nucleotides for sequence identification, but does not include allele-specific primer extension for enrichment of one allele over the other for further analysis of the allele of interest as part of the kit. WO Patent Application No. 2003100101 describes isolation of one sequence in a mixture by hybridization markers and single-strand specific nucleases for use in single-molecule analysis. U.S. Patent Application Publication No. 20030039964 describes a method for isolation of one sequence in a mixture by hybridization to a fixed probe, but does not disclose the use of reverse transcription. U.S. Patent No. 6,013,431 describes a method for analysis of bases adjacent to a hybridized, immobilized oligo, but does not disclose enrichment of one allele over the other. WO Patent Application No. 9820166 describes a method for specific selection of one allele over the other, followed by mass spectroscopic analysis of the selected molecule, but does not disclose the use of reverse transcription. None of these references disclose methods and diagnostic kits for linking polymorphic sequences to expanded repeat mutations for improved allele-specific diagnosis.

U.S. Patent Publication No. 20040241854 (Davidson) discloses allele-specific inhibition of specific single nucleotide polymorphism variants, and presents data showing that "expanded CAG repeats and adjacent sequences, while accessible to RNAi, may not be preferential targets for silencing" thus describing the problem that our invention addresses (determining what SNP variant at a remote molecular position is linked to the expanded CAG repeat in a particular patient), but does not teach the use of reverse transcription using an allele-specific primer to solve this problem, nor otherwise disclose a method for how to solve this problem. U.S. Patent Publication No. 20060270623 (McSwiggen) discloses multiple siRNA sequences, including those comprising SNP variants, but does not provide any working examples regarding allele-specific RNA interference using these disclosed siRNA sequences, nor disclose how to determine which allele-specific siRNA to administer to a particular Huntington's disease human patient in order to effectively treat that patient's disease by suppression of only the expanded Huntington allele in that patient.

Accordingly, there is need in the art for novel compounds, methods, and kits for allele-specific diagnostics and therapies.

### SUMMARY OF THE INVENTION

Applicants have invented methods and kits for determining which variant allele of a single nucleotide polymorphism ("SNP") located at a distance from a disease-causing mutation co-segregates with the disease-causing mutation. In other words, the invention will allow for the determination of which SNP allele is located on the same mRNA transcript as the transcribed disease-causing mutation.

In one aspect, the invention provides a method for determining which single nucleotide polymorphism variant of an allele from a gene isolated from a heterozygous patient is on the same mRNA transcript as a disease-causing mutation at a remote region of the gene's mRNA comprising: a) an allele-specific reverse transcription reaction using an allele-specific primer which recognizes one single nucleotide polymorphism variant, and b) analysis of an allele-specific cDNA product from the allele-specific reverse transcription reaction at the remote region of the gene to determine the presence or absence of the mutation on the allele-specific cDNA product, wherein the allele-specific primer is shorter than about 20 nucleotides. In one embodiment, the 3' end of the allele-specific primer is positioned at the single nucleotide polymorphism nucleotide position.

In another aspect, the invention provides a method of treating a patient comprising determining which SNP variant is on the same mRNA transcript as a disease-causing mutation according to the method recited above, and applying an allele-specific therapy to the SNP variant, wherein the allele-specific therapy comprises an siRNA comprising a double-stranded portion, wherein the single nucleotide polymorphism site is located within seven nucleotides from an end of the double stranded portion. The allele-specific therapy of the present invention includes by way of example allele-specific RNA interference using siRNA or shRNA.

In yet another aspect, the invention provides a kit for determining which single nucleotide polymorphism variant of an allele of a heterozygous patient is on the same mRNA transcript as a disease-causing mutation located at a remote region of the gene's mRNA comprising a) an allele-specific primer which recognizes one single nucleotide polymorphism variant, and b) a set of instructions, wherein the allele-specific primer is shorter than about 20 nucleotides. In one embodiment, the 3' end of the allele-specific primer is positioned at the single nucleotides polymorphism nucleotide position.

### BRIEF DESCRIPTION OF FIGURES

FIG. 1 is a schematic illustration of allele-specific reverse transcription for the "A" allele.
FIG. 2 is a photograph of a gel showing results of allele-specific reverse transcription.
FIG. 3 is an illustration of primers and molecular beacons.
FIG. 4 is an illustration of allele specificity of the molecular beacons used for the allele-specific quantification of real-time PCR product
FIG. 5 illustrates that a siRNA molecule designated as 363125_C-16 inhibits the mutant, but not the wild-type huntingtin mRNA.

### DETAILED DESCRIPTION OF THE INVENTION

The present invention relates to methods and kits for performing allele-specific reverse transcription from an SNP site and analysis of a cDNA at a region of gene mutation. The methods, systems and reagents of the present invention are applicable to any disease which contains an SNP variant of an allele in a heterozygous subject that is on the same mRNA transcript as a disease-causing mutation that is at a remote region of the gene's mRNA.

To aid in the understanding of the invention, the following non-limiting definitions are provided:
The term "gene" refers to a DNA sequence that comprises control and coding sequences necessary for the production of a polypeptide or its precursor. The polypeptide can be encoded by a full length coding sequence (either genomic DNA or cDNA) or by any portion of the coding sequence so long as the desired activity is retained. In some aspects, the term "gene" also refers to an mRNA sequence or a portion thereof that directly codes for a polypeptide or its precursor.

The term "transfection" refers to the uptake of foreign DNA by a cell. A cell has been "transfected" when exogenous (i.e., foreign) DNA has been introduced inside the cell membrane. Transfection can be either transient (i.e., the introduced DNA remains extrachromosomal and is diluted out during cell division) or stable (i.e., the introduced DNA integrates into the cell genome or is maintained as a stable episomal element).

"Cotransfection" refers to the simultaneous or sequential transfection of two or more vectors into a given cell.

The term "promoter element" or "promoter" refers to a DNA regulatory region capable of being bound by an RNA polymerase in a cell (e.g., directly or through other promoter-bound proteins or substances) and initiating transcription of a coding sequence. A promoter sequence is, in general, bounded at its 3' terminus by the transcription initiation site and extends upstream (5' direction) to include the minimum number of bases or elements necessary to initiate transcription at any level. Within the promoter sequence may be found a transcription initiation site (conveniently defined, for example, by mapping with nuclease S1), as well as protein binding domains (consensus sequences) responsible for the binding of RNA polymerase. The promoter may be operably associated with other expression control sequences, including enhancer and repressor sequences.

The term "in operable combination", "in operable order" or "operably linked" refers to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced.

The term vector" refers to a nucleic acid assembly capable of transferring gene sequences to target cells (e.g., viral vectors, non-viral vectors, particulate carriers, and liposomes). The term "expression vector" refers to a nucleic acid assembly containing a promoter which is capable of directing the expression of a sequence or gene of interest in a cell. Vectors typically contain nucleic acid sequences encoding selectable markers for selection of cells that have been transfected by the vector. Generally, "vector construct," "expression vector," and "gene transfer vector," refer to any nucleic acid construct capable of directing the expression of a gene of interest and which can transfer gene sequences to target cells. Thus, the term includes cloning and expression vehicles, as well as viral vectors.

The term "antibody" refers to a whole antibody, both polyclonal and monoclonal, or a fragment thereof, for example a F(ab)₂, Fab, FV, VH or VK fragment, a single chain antibody, a multimeric monospecific antibody or fragment thereof, or a bi- or multi-specific antibody or fragment thereof. The term also includes humanized and chimeric antibodies.

The term "treating" or "treatment" of a disease refers to executing a protocol, which may include administering one or more drugs to a patient (human or otherwise), in an effort to alleviate signs or symptoms of the disease. Alleviation can occur prior to signs or symptoms of the disease appearing, as well as after their appearance. Thus, "treating" or "treatment" includes "preventing" or "prevention" of disease. In addition, "treating" or "treatment" does not require complete alleviation of signs or symptoms, does not require a cure, and specifically includes protocols which have only a marginal effect on the patient.

The term "patient" refers to a biological system to which a treatment can be administered. A biological system can include, for example, an individual cell, a set of cells (e.g., a cell culture), an organ, a tissue, or a multicellular organism. A patient can refer to a human patient or a non-human patient.

The terms "remote region" or "remote location" indicate a distance of at least 100 base pairs from the SNP site to the site of the disease-causing mutation, such as, for example, at least 0.5 kb, or at least 1 kb, or at least 2 kb or at least 3 kb, or at least 4 kb or at least 5 kb, or at least 6 kb or more.

The term "practitioner" refers to a person who uses methods, kits and compositions of the current invention on the patient. The term includes, without limitations, doctors, nurses, scientists, and other medical or scientific personnel.

The terms "siRNA molecule," "shRNA molecule," "RNA molecule," "DNA molecule," "cDNA molecule" and "nucleic acid molecule" are each intended to cover a single molecule, a plurality of molecules of a single species, and a plurality of molecules of different species.

The term "siRNA" refers to a double-stranded RNA molecule wherein each strand is between about 15 and about 30 bases of ribonucleic acid in length, and the two strands have a region of complementarity such that the two strands hybridize or "base pair" together through the annealing of complementary bases (Adenosine to Uracil, and Guanine to Cytosine). For some siRNA molecules, the two strands hybridize together in a manner such that there is an overhang of non-annealed bases at the 5' or 3' ends of the strand. For other siRNA molecules, the two strands hybridize together such that each base of one strand is paired with a base of the other strand. For some siRNA molecules, the two strands may not be 100% complementary, but may have some bases that do not hybridize due to a mismatch. For some siRNA molecules, the RNA bases may be chemically modified, or additional chemical moieties may be conjugated to one or more ends of one or more of the strands.

The term "shRNA" refers to a "short, hairpin" RNA molecule comprised of a single strand of RNA bases that self-hybridizes in a hairpin structure. The RNA molecule is comprised of a stem region of RNA bases that hybridize together to form a double-stranded region, and a loop region of RNA bases that form the bend of the hairpin. The term "shRNA" also refers to a DNA molecule from which a short, hairpin RNA molecule may be transcribed *in vitro* or *in vivo.*

The methods of the present invention utilize routine techniques in the field of molecular biology. Basic texts disclosing general molecular biology methods include Sambrook et al., Molecular Cloning, A Laboratory Manual (3d ed. 2001) and Ausubel et al., Current Protocols in Molecular Biology (1994).

The present invention relates generally to compositions and methods for diagnosing diseases which have an allele-specific therapy and a disease-causing mutation that is sufficiently distant from the molecular site of the therapy. Table 1 depicts certain diseases applicable to the present invention. Table 1 was derived from information previously published (DiProspero (2005)). Table 1 describes in part examples of triplet repeat expansion diseases and the mutant gene associated with each disease.

**Table 1**

| Triplet Repeat Expansion Disorders | | | | |
|---|---|---|---|---|
| Non-coding repeats | | | | |
| Disease | Symptoms | Gene | Locus | Protein |
| Dystropbia myotonica 1 | Weakness, Myotonia | *DMPK* | 19q13 | Dystrophia myotonica Protein kinase |
| Spinocerebellar ataxia 8 | Ataxia | *Antisense* to *KLHL1* | 13q21 | Undetermined |
| Huntington disease-like2 | Chorea, dementia | *JPH3* | 16q24.3 | Junctophilin 3 |

| Polyglutamine disorder | | | | |
|---|---|---|---|---|
| Spinal and bulbar muscular atrophy | Weakness | *AR* | Xq13-q21 | Androgen receptor |
| Huntington disease | Chorea, dementia | *IT15* | 4P16.3 | Huntingtiin |
| Dentatorubral-pallidoluysian atrophy | Ataxia, myoclonic epilepsy, dementia | *DRPLA* | 12p13.31 | Atrophin 1 |
| Spinocerebellar ataxia 1 | Ataxia | *SCA1* | 6p23 | Ataxin 1 |
| Spinocerebellar ataxia 2 | Ataxia | *SCA2* | 12q24.1 | Ataxin 2 |
| Spinocerebellar ataxia 3 (Machado-Joseph disease) | Ataxia | *SCA3*/*MJD* | 14q32.1 | Ataxin 3 |
| Spinocerebellar ataxia 6 | Ataxia | *CACNAlA* | 19pl3 | α_{1A}-voltage-dependent calcium channel subunit |
| Spinocerebellar ataxia 7 | Ataxia | *SCA7* | 3p12-p13 | Ataxin 7 |
| Spinocerebellar ataxia 17 | Ataxia | *TBP* | 6q27 | TATA box binding protein |

| Polyalanine disorders* | | | | |
|---|---|---|---|---|
| oculopharyageal dystrophy | Weakenas | *PABPR1* | 1aq11.2-q13 | Poly(A)-binding protein 2 |
| Congential central | Respiratory | *PHOX2B* | 4pl2 | Paired-like |
| hypoventilation syndrome | difficulties | | | homeobox 2B |
| Infantile spasms | Mental retardation, epilepay | ARX | Xp22.13 | Aristalese-related homeobox, X-linked |
| Synpolydactyly | Limb malformation | *HOXD13* | 2q3l-q32 | Homeobox D13 |

*Polyalanine expansions have also been reported among mutations in other genes, including RUNX2 (runt-related transcription factor2) in cleidocranial dysplasia, ZIC2 (Zic family member 2) in holoprosencephaly HOXA13 (homeobox A13) in hand-foot-genital syndrome, and FOXL2 (forkhead box L2) in type II blepharophimosis, ptosis, and epicanthus inversus syndrome. Small aspartic acid repeat expansions have been reported among other mutations in the COMP (cartilage oligomeric mat4rix protein) gene in patients with multiple epiphyseal dysplasia.

The present invention is not limited to the diseases described above. There may be situations where a disease is caused by many different mutations in a single gene (thus designing many different gene-targeting therapies may not be practical from a commercial perspective). However, if one or two expressed SNPs are present in the disease-associated gene, then the SNPs may actually serve as the molecular target for the therapy (and thus determination of linkage of the SNP to the disease-causing mutation would be essential).

For purposes of illustration, only HD will be discussed herein as an example of a triplet repeat expansion disease and example of the applicability of the present invention in providing methods and kits for determining allele-specific reverse transcription from an SNP site and analysis of a cDNA at a region of mutation.

The coding region of the *IT15* gene is about 13,000 bases long. The HD disease-causing mutation is the expansion of the CAG repeat region. The CAG repeat region starts at about nucleotide position 15. If the CAG triplets repeat for about 25 or 30 times, the patient is not at risk of the disease. If however, more than 37 CAG repeats occur in a row on the nucleotide sequence then the patient is going to get Huntington's disease.

About ten thousand bases downstream from the CAG repeat sequence, there is a natural variation (Single Nucleotide Polymorphism, or SNP) of the *IT15* gene in the human population, where for many people it might be an "A residue" and for many others it is a "C residue". That is just a normal variation, as it does not cause any disease. The information about the SNP can be used to determine that a child of a Huntington's disease patient has inherited an allele with the "A residue" from one parent and an allele with the "C residue" from the other parent.

The practitioner also knows that one of the patient's parents has HD and would like to know if the patient will also get HD. The practitioner can actually determine whether the patient is going to get HD or not, by looking at both of the patient's *IT15* alleles, and determining how many CAG repeats the gene contains. If one of the CAG repeats is longer than 37, then the patient will get HD. Further, the practitioner can determine whether the patient is heterozygous (i.e., one allele has a normal number of repeats, e.g., 20, while the other allele has expanded repeats, e.g., 37). Analyzing the *IT15* gene downstream of the CAG repeats, the practitioner may find that the patient received a "C residue" from one parent and an "A residue" from the other parent. Thus, the crucial issue for the allele-specific diagnosis is which SNP is on the same mRNA transcript as the expanded number of repeats in the patient's *IT15* gene. Isolating the genetic information from the patient's parents may not help because it is possible that one or both parents are also heterozygous (e.g., each parent has two SNP variants of the gene (i.e., an A residue and a C residue variants). This disclosure provides a method of determining which SNP allele of the gene co-segregates with the disease-causing mutation.

One aspect of the present invention provides a diagnostic test, allowing the practitioner to determine which allele, classified by the nucleotide at the SNP position, co-segregates with the disease-causing mutation. In one embodiment, the test comprises a method for determining which single nucleotide polymorphism variant of an allele from a gene isolated from a heterozygous patient is on the same mRNA transcript as a disease-causing mutation at a remote region of the gene's mRNA comprising: a) an allele-specific reverse transcription reaction using an allele-specific primer which recognizes one single nucleotide polymorphism variant, wherein the 3' end of the allele-specific primer is positioned at the single nucleotide polymorphism nucleotide position, and b) analysis of an allele-specific cDNA product from the allele-specific reverse transcription reaction at the remote region of the gene to determine the presence or absence of the mutation on the allele-specific cDNA product. The inventors have discovered that the primer should preferably be shorter than about 20 nucleotides, e.g., about 15 nucleotides, long, because of a possibility that primers which are longer than about 20 nucleotides will not discriminate between the targeted SNP variants.

In a layman's terms, the practitioner takes RNA from the patient and applies a reverse transcription primer that recognizes just the "A allele." The "A allele" specific primer will have at its 3' position a complement to the SNP variant of interest. In case of the A-variant, the "A allele" specific primer will have the T at the 3' end, and so when this "A allele" specific primer anneals to the mRNA, it will base-pair with the 3' end and allow the reverse transcriptase to proceed to synthesize the cDNA from the "A allele." Conversely, the "A allele" primer will not base-pair at the 3' end of the primer with the "C allele" (since T is not complementary to C). Thus, the reverse transcription polymerase will not be able to produce cDNA from the C allele. On the other hand, in the "A" portion of a reaction, the practitioner will obtain a pool of the cDNAs that corresponds to the "A allele." The reaction can be repeated in a separate tube with a "C allele" specific primer and no "A allele" primer. A person of ordinary skill in the art will understand that the "C allele" specific primer will have a G on its 3' end. Essentially the practitioner will perform at least one allele-specific reverse transcription reaction, but preferably two allele-specific reverse transcriptions reactions (each with its own allele-specific primer), on the mRNA from the patient. As a result, the practitioner will have two sub-populations of cDNA, wherein each subpopulation is allele-specific, and the practitioner knows which pool corresponds to which variant. Thus, the practitioner will be able to use any number of possible methods, the simplest being PCR to analyze the upstream portion of the cDNA containing the CAG repeat region and quantify the number of the repeats from the cDNA products that came specifically from the "C reaction" or specifically from the "A reaction."

The embodiment of the invention described above employs the notion that a mismatch on the 3' position of the allele-specific primer will not allow reverse transcriptase to produce cDNA from the allele with a mismatched SNP variant. A person of ordinary skill in the art will undoubtedly recognize that the 3' end of the allele-specific primer does not have to be positioned at the single nucleotide polymorphism nucleotide position. For example, a skilled artisan may design primers and conditions of the reverse transcription reaction in such a way that the allele-specific primer will not bind altogether and thus lead to the same end result: absence of cDNA the allele with a mismatched SNP variant.

The accurate determination of the number of CAG repeats is required for the DNA-based predictive testing of at-risk individuals. To date, CAG repeat length determination is based on polymerase chain reaction (PCR) amplification of genomic DNA using primers flanking the CAG repeat region in the *IT15* gene, and subsequent electrophoretic separation of the products in denaturing polyacrylamide gels (Williams et al., (1999) Comparative semi-automated analysis of (CAG) repeats in the HD gene: use of internal standards. Mol. Cell Probes, 13:283-289).

Numerous methods and commercial kits for the synthesis of first strand cDNA molecules are well known in the art. Examples include the Superscript^{™} Double Strand cDNA Synthesis Kit (Invitrogen, Carlsbad, CA), the Array 50^{™}, Array 350^{™} and Array 900^{™} Detection Kits (Genisphere, Hatfield, PA), and the CyScribe^{™} Post-Labelling Kit (Amersham, Piscataway, NJ). RNA molecules (e.g., mRNA, hnRNA, rRNA, TRUA, miRNA, snoRNA, non-coding RNAs) from a source of interest are used as templates in a reverse transcription reaction. The RNA may be obtained from a mammalian or more preferably human tissue or cell source. The methods of the present invention are particularly suited for amplification of RNA from small numbers of cells, including single cells, which can be purified from complex cellular samples using, e.g., micromanipulation, fluorescence-activated cell sorting (FACS) and laser microdissection techniques (see Player et al., Expert Rev. Mol. Diagn. 4:831 (2004)).

Any reverse transcriptase can be used in the initial reverse transcription reaction, including thermostable, RNAse H⁺ and RNase H⁻ reverse transcriptases. Preferably, an RNase H⁻ reverse trancriptase is used.

Primers for first strand cDNA synthesis can be obtained commercially or synthesized and purified using techniques well known in the art. As disclosed above, the inventors discovered that primers which are about 15 nucleotides long provide the best results in terms of discriminating between the SNP variants and efficiency in producing the allele-specific cDNA.

PCR amplifications of the CAG repeat region have primarily been performed by incorporating [α-³²P]dNTPs, or using ³²_{P} or fluorescently end-labeled primers. Sizing of fluorescently end-labeled amplification products was performed in various Applied Biosystems DNA sequencers (Andrew et al., (1993) Nature Genet., 4:398-403; Choudhry et al., (2001) Hum. Mol. Genet., 10:2437-2446; Ishii et al., (2001) J. Clin. Endocrinol. Metab., 86:5372-5378; Le et al., (1997) Mol. Pathol., 50:261-265; Mangiarini et al., (1997) Nature Genet., 15:197-200; Pelotti et al., (2001) Am. J. Forensic Med. Pathol., 22:55-57; Wallerand et al., (2001) Fertil. Steril., 76:769-774; Warner et al., (1993) Mol. Cell Probes, 7:235-239; and Warner et al., (1996) J. Med. Genet., 33:1022-1026).

High-resolution method can be used for the exact length determination of CAG repeats in HD genes as well as in genes affected in related CAG repeat disorders (Elisabeth Möncke-Buchner et al., Nucleic Acids Res. 2002 August 15; 30(16)).

A wide variety of kits may be prepared according to present invention. For example, a kit may include a single stranded promoter template comprising at least one RNA polymerase recognition sequence; and instructional materials for synthesizing cDNA molecules using said promoter template. While the instructional materials typically comprise written or printed materials, they are not limited to such. Any medium capable of storing such instructions and communicating them to an end user is contemplated by this invention. Such media include, but are not limited to, electronic storage media (e.g., magnetic discs, tapes, cartridges, chips), optical media (e.g., CD ROM), and the like. Such media may include addresses to internet sites that provide such instructional materials.

The kits of the present invention may further include one or more of the following components or reagents: a reverse transcriptase (preferably with DNA-dependent DNA polymerase activity); an RNase inhibitor; an enzyme for attaching a 3' oligodeoxynucleotide tail onto DNA molecules (e.g., terminal deoxynucleotidyl transferase); an enzyme for degrading RNA in RNA/DNA duplexes (e.g., RNase H); and one or more RNA polymerases (e.g., T7, T3 or SP6 RNA polymerase). Additionally, the kits may include buffers, primers (e.g., oligodT primers, random primers), nucleotides, labeled nucleotides, an RNase inhibitor, polyA polymerase, RNase-free water, containers, vials, reaction tubes, and the like compatible with the synthesis of sRNA molecules according to the methods of the present invention. The components and reagents may be provided in containers with suitable storage.media.

A person of ordinary skill in the art will appreciate that such allele-specific diagnosis empowers a practitioner to devise and implement an allele-specific treatment which generally comprises inactivation of the mutated copy of the gene. It is known that patients are able to survive and live healthy lives with only one functioning copy of the HD gene. It is known that the expression of the mutant gene is causing the trouble for the HD patient. Applicants' therapeutic model provides for selectively shutting off mutant gene expression without affecting expression of the normal gene, and is applicable to any disease which contains an SNP variant of an allele in a heterozygous subject that is on the same mRNA transcript as a disease-causing mutation that is at a remote region of the gene's mRNA.

Accordingly, another aspect of the present invention provides a method of treating a patient susceptible to Huntington's disease comprising: a) determining which single nucleotide polymorphism variant is on the same mRNA transcript as a disease-causing mutation according to an allele-specific reverse transcription reaction using an allele-specific primer which recognizes one single nucleotide polymorphism variant, wherein further the 3' end of the primer is positioned at the single nucleotide polymorphism nucleotide position, and b) analysis of the resulting cDNA product from the reverse transcription reaction at the region of the mutation to determine the presence or absence of the mutation on this allele-specific cDNA product, and c) applying an allele-specific therapy to the SNP variant. In one embodiment, the allele-specific therapy comprises an RNA molecule comprising a double-stranded portion, wherein the single nucleotide polymorphism site is located within seven nucleotides from an end of the double stranded portion. In one embodiment, the double stranded portion is between 15 and 23 nucleotides long, e.g., about 19 nucleotides long. Further, as discussed above, the siRNA molecule may contain a loop (e.g., shRNA), a 3' overhand or a 5' overhang which are outside of the double-stranded portion. The instant invention also provides a method of allele-specific therapy, wherein the double stranded portion does not contain a mismatch in a position adjacent to the single nucleotide polymorphism site. Thus, in one embodiment of the invention, the siRNA molecule does not contain any mismatches and one strand of the double-stranded portion is 100% identical to the portion of the targeted mRNA transcript. In one embodiment, wherein the disease treated by the allele-specific therapy is Huntington's disease, the non-limiting example of a single nucleotide polymorphism site suitable for the allele-specific therapy is rs262125.

It should be noted that the allele-specific therapy could itself operate at a different SNP site than the SNP site used to make the determination about which allele contains the mutation, so long as the SNP site of the therapy target and the SNP site used to identify the mutation-containing allele are already determined, before the therapy is administered to the patient, to be linked; that is, on the same mRNA transcript.

In some embodiments of the present invention the allele-specific therapy comprises allele-specific RNA interference using siRNA or shRNA. In this embodiment of the invention, the allele-specific therapy destroys the "A allele" of the patient. In this embodiment the siRNA targets the "A allele" upon introduction into the subject's brain by any method known to those of skill in the art (See for example, U.S. Application No. 11/253,393, U.S. Application No. 10/852,997, U.S. Application No. 10/721,693, U.S. Application No. 11/157,608, and PCT Patent Application No. US05/022156, which are incorporated herein in their entirety). When the siRNA is delivered into a cell it is used by proteins in the cell (known as the RISC complex) to find and destroy the mRNA from the Huntington's gene that has the "A allele." Thus, the messenger RNA is destroyed before it can be used to make protein. Conversely, the allele that came from the healthy parent does not get destroyed and so its messenger RNA still survives to be used to make functional biologically active protein.

The design and use of small interfering RNA complementary to mRNA targets that produce particular proteins is a recent tool employed by molecular biologists to prevent translation of specific mRNAs. Various groups have been recently studying the effectiveness of siRNAs as biologically active agents for suppressing the expression of specific proteins involved in neurological disorders. Caplen et al. Human Molecular Genetics, 11(2): 175-184 (2002) assessed a variety of different double stranded RNAs for their ability to inhibit cell expression of mRNA transcripts of the human androgen receptor gene containing different CAG repeats. Their work found gene-specific inhibition occurred with double stranded RNAs containing CAG repeats only when flanking sequences to the CAG repeats were present in the double stranded RNAs. They were also able to show that constructed double stranded RNAs were able to rescue caspase-3 activation induced by expression of a protein with an expanded polyglutamine region. Xia, Mao, et al., Nature Biotechnology, 20: 1006-1010 (2002) demonstrated the inhibition of polyglutamine (CAG) expression in engineered neural PC12 clonal cell lines that express a fused polyglutamine-fluorescent protein using constructed recombinant adenovirus expressing siRNAs targeting the mRNA encoding green fluorescent protein.

One aspect of the present invention provides an siRNA molecule corresponding to at least a portion of a gene containing an SNP variant of an allele in a heterozygous subject that is on the same mRNA transcript as a disease-causing mutation located at a remote region of the gene's mRNA, wherein such siRNA nucleic acid sequence is capable of inhibiting expression of the mRNA transcript containing the disease-causing mutation in a cell. siRNAs are typically short (19-29 nucleotides), double-stranded RNA molecules that cause sequence-specific degradation of complementary target mRNA known as RNA interference (RNAi). Bass, Nature 411:428 (2001).

Accordingly, in some embodiments, the siRNA molecules comprise a double-stranded structure comprising a sense strand and an antisense strand, wherein the antisense strand comprises a nucleotide sequence that is complementary to at least a portion of a desired nucleic acid sequence and the sense strand comprises a nucleotide sequence that is complementary to at least a portion of the nucleotide sequence of said antisense region, and wherein the sense strand and the antisense strand each comprise about 19-29 nucleotides.

Any desired nucleic acid sequence can be targeted by the siRNA molecules of the present invention. Nucleic acid sequences encoding desired gene targets are publicly available from Genbank.

The siRNA molecules targeted to desired sequence can be designed based on criteria well known in the art (e.g., Elbashir et al., EMBO J. 20:6877 (2001)). For example, the target segment of the target mRNA preferably should begin with AA (most preferred), TA, GA, or CA; the GC ratio of the siRNA molecule preferably should be 45-55%: the siRNA molecule preferably should not contain three of the same nucleotides in a row; the siRNA molecule preferably should not contain seven mixed G/Cs in a row; the siRNA molecule preferably should comprise two nucleotide overhangs (preferably TT) at each 3' terminus; the target segment preferably should be in the ORF region of the target mRNA and preferably should be at least 75 bp after the initiation ATG and at least 75 bp before the stop codon; and the target segment preferably should not contain more than 16-17 contiguous base pairs of homology to other coding sequences.

Based on some or all of these criteria, siRNA molecules targeted to desired sequences can be designed by one of skill in the art using the aforementioned criteria or other known criteria (e.g., Gilmore et al., J. Drug Targeting 12:315 (2004); Reynolds et al., Nature Biotechnol. 22:326 (2004); ; Ui-Tei et al., Nucleic Acids Res. 32:936 (2004)). Such criteria are available in various web-based program formats useful for designing and optimizing siRNA molecules (e.g., siDESIGN Center at Dharmacon; BLOCK-iT RNAi Designer at Invitrogen; siRNA Selector at Wistar Insitute: siRNA Selection Program at Whitehead Institute; siRNA Design at Integrated DNA Technologies; siRNA Target Finder at Ambion; and siRNA Target Finder at Genscript).

siRNA molecules targeted to desired sequences can be produced in vitro by annealing two complementary single-stranded RNA molecules together (one of which matches at least a portion of a desired nucleic acid sequence) (e.g., U.S. Pat. No. 6,506,559) or through the use of a short hairpin RNA (shRNA) molecule which folds back on itself to produce the requisite double-stranded portion (Yu et al., Proc. Natl. Acad. Sci. USA 99:6047 (2002)). Such single-stranded RNA molecules can be chemically synthesized (e.g., Elbashir et al., Nature 411:494 (2001)) or produced by *in vitro* transcription using DNA templates (e.g., Yu et al., Proc. Natl. Acad. Sci. USA 99:6047 (2002)). When chemically synthesized, chemical modifications can be introduced into the siRNA molecules to improve biological stability. Such modifications include phosphorothioate linkages, fluorine-derivatized nucleotides, deoxynucleotide overhangs, 2'-*O*-methylation. 2'-*O*-allylation, and locked nucleic acid (LNA) substitutions (Dorset and Tuschl, Nat. Rev. Drug Discov. 3:318 (2004); Gilmore et al., J. Drug Targeting 12:315 (2004)).

siRNA molecules targeted to desired target sequences can be introduced into cells to inhibit expression. Alternatively, DNA molecules from which shRNA molecules targeted to desired target sequences can be introduced into cells to inhibit expression. Accordingly, another aspect of the present invention provides for inhibiting expression of an mRNA sequence containing an SNP allele and a disease-causing mutation in a cell comprising introducing into a cell at least one siRNA molecule or shRNA molecule that corresponds to at least a portion of the mRNA nucleic acid sequence. Any cell can be targeted. For example, the siRNA or shRNA molecules are introduced into a heart cell or brain cell. In some embodiments, the brain cell is from a subject at risk for HD, i.e., the offspring of a HD patient.

The siRNA molecules produced herein can be introduced into cells in vitro or ex vivo using techniques well-known in the art, including electroporation, calcium phosphate co-precipitation, microinjection, lipofection, polyfection, and conjugation to cell penetrating peptides (CPPs). The siRNA molecules can also be introduced into cells in vivo by direct delivery into specific organs such as the liver, brain, eye, lung and heart, or systemic delivery into the blood stream or nasal passage using naked siRNA molecules or siRNA molecules encapsulated in biodegradable polymer microspheres (Gilmore et al., J. Drug Targeting 12:315 (2004)).

Alternatively, siRNA molecules targeted to specific mRNA sequences can be introduced into cells *in vivo* by endogenous production from an expression vector(s) encoding the sense and antisense siRNA sequences. Accordingly, another aspect of the present invention provides an expression vector comprising at least one DNA sequence encoding a siRNA molecule corresponding to at least a portion of a specific mRNA nucleic acid sequence capable of inhibiting expression of a specific mRNA in a cell operably linked to a genetic control element capable of directing expression of the siRNA molecule in a cell. Expression vectors can be transfected into cells using any of the methods described above.

Genetic control elements include a transcriptional promoter, and may also include transcription enhancers to elevate the level of mRNA expression, a sequence that encodes a suitable ribosome binding site, and sequences that terminate transcription. Suitable eukaryotic promoters include constitutive RNA polymerase II promoters (e.g., cytomegalovirus (CMV) promoter, the SV40 early promoter region, the promoter contained in the 3' long terminal repeat of Rous sarcoma virus (RSV), the herpes thymidine kinase (TK) promoter, and the chicken beta-actin promoter), cardiac- tissue- specific RNA polymerase II promoters (e.g., the ventricular myosin light chain 2 (MLC-2v) promoter, and the sodium-calcium exchanger gene H1 promoter (NCXiHI)), and RNA polymerase III promoters (e.g., U6, Hl, 7SK and 7SL).

In some embodiments, the sense and antisense strands of siRNA molecules are encoded by different expression vectors (i.e., cotransfected) (e.g., Yu et al., Proc. Natl. Acad. Sci. USA 99:6047 (2002). In other embodiments, the sense and antisense strands of siRNA molecules are encoded by the same expression vector. The sense and antisense strands can be expressed separately from a single expression vector, using either convergent or divergent transcription (e.g., Wang et al., Proc. Natl. Acad. Sci. USA 100:5103 (2003); Tran et al., BMC Biotechnol. 3:21 (2003)). Alternatively, the sense and antisense strands can be expressed together from a single expression vector in the form of a single hairpin RNA molecule, either as a short hairpin RNA (shRNA) molecule (e.g., Arts et al., Genome Res. 13:2325 (2003)) or a long hairpin RNA molecule (e.g., Paddison et al., Proc. Natl. Acad. Sci. USA 99:1443 (2002)).

Although numerous expression vectors can be used to express siRNA molecules in cells (Dorsett and Tuschl, Nat. Rev. Drug Discov.3:318 (2004)), viral expression vectors are preferred, particularly those that efficiently transduce heart cells (e.g., alphaviral, lentiviral, retroviral, adenoviral, adeno-associated viral (AAV)) (Williams and Koch, Annu. Rev. Physiol. 66:49 (2004); del Monte and Hajjar, J. Physiol. 546. 1: 49 (2003). Both adenoviral and AAV vectors have been shown to be effective at delivering transgenes (including transgenes directed to deseases) into heart, including failing cardiomycoytes (e.g., Iwanaga et al., J. Clin. Invest. 113:727 (2004); Seth et al., Proa. Natl. Acad. Sci. USA 101:16683 (2004); Champion et al., Circulation 108:2790 (2003); Li et al., Gene Ther. 10:1807 (2003) ; Vassalli et al., Int. J. Cardiol. 90:229 (2003); del Monte et al., Circulation 105:904 (2002); Hoshijima et al., Nat. Med. 8:864 (2002); Eizema et al., Circulation 101:2193 (2000); Miyamoto et al., Proc. Natl. Acad. Sai. USA 97:793 (2000); He et al., Circulation 100:974 (1999). Recent reports have demonstrated the use of AAV vectors for sustained gene expression in mouse and hamster myocardium and arteries for over one year (Li et al., Gene Ther. 10:1807 (2003); Vassalli et al., Int. J. Cardiol. 90:229 (2003)). In particular, expression vectors based on AAV serotype 6 have been shown to efficiently transduce both skeletal and cardiac muscle (e.g., Blankinship et al., Mol. Ther. 10:671 (2004)). The present invention also provides for the use of coxsackie viral vectors for delivery of desired siRNA sequences.

Following introduction of the desired siRNA molecules into cells, changes in desired gene product levels can be measured if desired. Desired gene products include, for example, desired mRNA and desired polypeptide, and both can be measured using methods well-known to those skilled in the art. For example, desired mRNA can be directly detected and quantified using, e.g., Northern hybridization, in situ hybridization, dot and slot blots, or oligonucleotide arrays, or can be amplified before detection and quantitation using, e.g., polymerase chain reaction (PCR), reverse-transcription-PCR (RT-PCR), PCR-enzyme-linked immunosorbent assay (PCR-ELISA), or ligase chain reaction (LCR).

Desired polypeptide (or fragments thereof) can be detected and quantified using various well-known immunological assays, such as, e.g., enzyme-linked immunosorbent assay (ELISA), radioimmunoassay (RIA), immunoprecipitation, immunofluorescence, and Western blotting. Anti-desired antibodies (preferably anti-human desired) for use in immunological assays are commercially available from, e.g., EMD Biosciences (San Diego, CA), Upstate (Charlottesville, VA), Abcam (Cambridge, MA), Affinity Bioreagents (Golden, CO) and Novus Biologicals (Littleton, CO), or may be produced by methods well-known to those skilled in the art.

The present invention relates to:
1. A method for determining which single nucleotide polymorphism variant of an allele from a gene isolated from a heterozygous patient is on the same mRNA transcript as a disease-causing mutation at a remote region of the gene's mRNA comprising: a) an allele-specific reverse transcription reaction using an allele-specific primer which recognizes one single nucleotide polymorphism variant, and b) analysis of an allele-specific cDNA product from the allele-specific reverse transcription reaction at the remote region of the gene to determine the presence or absence of the mutation on the allele-specific cDNA product, wherein the allele-specific primer is shorter than about 20 nucleotides.
2. The method of item 1, wherein the 3' end of the allele-specific primer is positioned at the single nucleotide polymorphism nucleotide position.
3.A method of item 1 or item 2 further comprising c) at least a second allele-specific reverse transcription reaction using at least a second allele-specific primer which recognizes at least a second single nucleotide polymorphism variant, and d) analysis of at least a second allele-specific cDNA product from at least the second allele-specific reverse transcription reaction at the remote region of the gene to determine the presence or absence of the mutation on at least the second allele-specific cDNA product.
4. The method of item 3 wherein the 3' end of at least the second allele-specific primer is positioned at the single nucleotide polymorphism nucleotide position.
5. A method of treating a patient comprising: a) determining which single nucleotide polymorphism variant is on the same mRNA transcript as a disease causing mutation according to the method of item 1, and b) applying an allele-specific therapy to the single nucleotide polymorphism variant.
6. The method of item 5 wherein the allele-specific therapy comprises an RNA molecule comprising a double-stranded portion, and the single nucleotide polymorphism site is located within seven nucleotides from an end of the double stranded portion.
7. The method of item 5 or 6, wherein the allele- specific therapy comprises allele-specific RNA interference using siRNA or shRNA.
8. The method of any one of items 5 to 7, wherein the patient is at risk of developing a neurodegenerative disorder selected from the group consisting of Dystophia myotonica, Spinocerebellar ataxia type 1, Spinocerebellar ataxia type 2, Spinocerebellar ataxia type 3, Spinocerebellar ataxia type 6, Spinocerebellar ataxia type 7, Spinocerebellar ataxia type 8, Spinocerebellar ataxia type 17, Huntington disease-like 2, Spinal and bulbar muscular atrophy, Huntington' s disease, Dentatorubral-pallidoluysian atrophy, Oculopharyngeal dystrophy, Congenital central hypoventilation syndrome, Infantile spasms, Synpolydactyly, Cleidocranial dysplasia, Holoprosencephaly, Hand-foot-genital syndrome, Type II blephorophimosis, ptosis, and epicanthus inversus syndrome. allele-specific therapy operates at the same single nucleotide polymorphism site used to determine which mRNA transcript contains the disease-causing allele in said patient.
9. The method of any one of items 5 to 8, wherein the allele-specific therapy operates at a different single nucleotide polymorphism site than the site used to determine which mRNA transcript contains the disease-causing allele in said patient.
10. The method of any one of items 5-9, wherein the double stranded portion is between 15 and 23 nucleotides long.
11. The method of any one of items 5-10, wherein the double stranded portion does not contain a mismatch in a position adjacent to the single nucleotide polymorphism site.
12. The method of any one of items 5-11, wherein one strand of the double-stranded portion is 100% identical to the portion of the targeted mRNA transcript.
13. The method of any one of items 8-12, wherein the disease is Huntington' s disease.
14. The method of items 13, wherein the single nucleotide polymorphism site is rs262125.
15. A kit for determining which single nucleotide polymorphism variant of an allele of a heterozygous patient is on the same mRNA transcript as a disease-causing mutation located at a remote region of the gene's mRNA comprising a) an allele-specific primer which recognizes one single nucleotide polymorphism variant, and b) a set of instructions.
16. The method of items 15, wherein the allele-specific primer is shorter than about 20 nucleotides.
17. The kit of item 15 or item 16, further comprising: at least a second allele-specific primer which recognizes at least a second single nucleotide polymorphism variant.
18. The kit of any one of items 15-17, wherein the 3' end of the allele-specific primer is positioned at the single nucleotide polymorphism nucleotide position.
19. The kit of any one of items 15-18, wherein the 3' end of the allele-specific primer is not positioned at the single nucleotide polymorphism nucleotide position.
20. The kit of any one of items 17-19, wherein the 3' end of at least the second allele-specific primer is positioned at the single nucleotide polymorphism nucleotide position.
21. The kit of any one of items 17-19, wherein the 3' end of at least the second allele-specific primer is not positioned at the single nucleotide polymorphism nucleotide position.
22. The kit of any one of items 15-21, wherein the allele-specific primer is selected from the group consisting Of SEQ. ID. 1 - SEQ. ID. 84.
23. The kit of any one of items 15-22, wherein at least the second allele-specific primer is selected from the group consisting of SEQ. ID. 11 - SEQ. ID. 20 if the allele-specific primer is selected from the group consisting of SBQ. ID. 1 - selected from the group consisting of SEQ. ID. 31 - SEQ, ID. 40 if the allele- specific primer is selected from the group consisting of SEQ. ID. 21 - SEQ. ID. 30; or at least the second allele-specific primer is selected, from the group consisting of SEQ. ID. 53 - SEQ. ID. 64 if the allele-specific primer is selected from the group consisting of SEQ. ID. 41 - SEQ. ID. 52; or at least the second allele-specific primer is selected from the group consisting of SEQ. ID. 75 - SEQ. ID. 84 if the allele-specific primer is selected from the group consisting of SEQ. ID. 65 - SEQ. ID. 74.
24. The kit of item 23, wherein the allele-specific primer is selected from the group consisting of SEQ. ID..42, SEQ. ID. 47, SEQ. ID. 52, SEQ. ID. 54, SEQ. ID. 59, and SEQ. ID. 64.

Specific embodiments according to the methods of the present invention will now be described in the following examples. The examples are illustrative only, and are not intended to limit the remainder of the disclosure in any way.

### EXAMPLES

### Example 1

*RNA-isolation and Reverse transcription reaction.* Applicants analyzed the CAG-repeat sequences in the Huntington's disease gene using the following allele-specific reverse transcription reaction. Table 2 defines the sequences for various allele-specific reverse transcription primers for use in determining which allele of a heterozygous patient's Huntington's disease gene contains the disease-causing allele, in accordance with the subject invention. The subject SNP sites in the Huntington's disease gene (IT15) are designated using the identification number provided by the National Center for Biotechnology Information (NCBI) database, accessible at: http://www.ncbi.nlm.nih.gov/entrez/query.fcgi?db=snp.

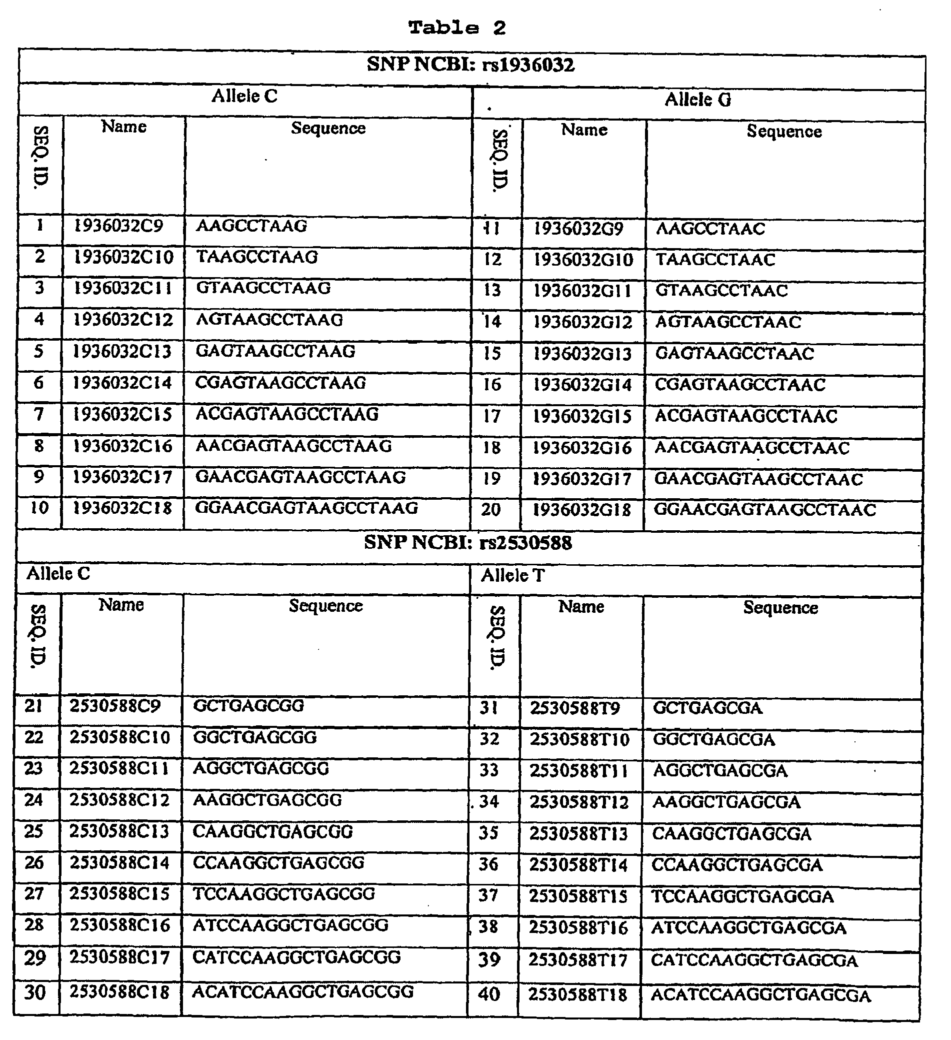

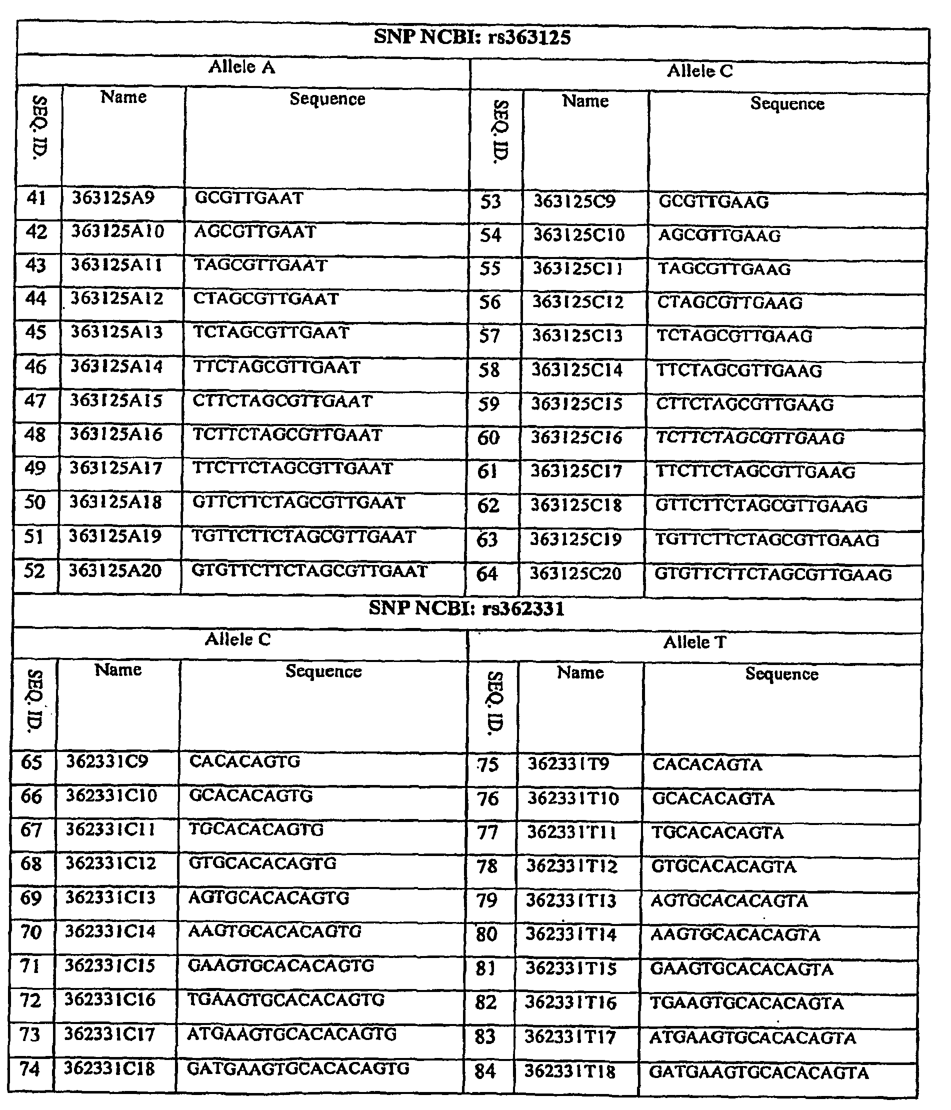

### Cell culture and genotyping.

Fibroblasts purchased from Coriell Cell Repositories (Camden, NJ) were cultured at 37°C with 5% CO₂ and maximum humidity. The growth medium was minimum essential medium containing 20% FBS, 1% PSN antibiotics, 1% fungizone, 2% non-essential amino acids, 2% amino acids and 2% vitamins (all reagents from Invitrogen). Genomic DNA was isolated using the DNeasy kit (Qiagen) and nucleotide identity for HD alleles at various SNP positions was determined by pyrosequencing conducted by Isogen Lifescience (The Netherlands).

### Identification of a heterozygous HD individual.

Fibroblasts from 21 individuals from different kindreds, diagnosed with HD or known to be at risk for HD, were purchased from Coriell Cell Repositories (Camden, NJ). DNA was harvested from each fibroblast culture and pyrosequenced to determine the nucleotide identity at eleven known single nucleotide polymorphism (SNP) sites within the protein coding region of the HD sequence. Heterozygosity was found at four of the eleven SNP sites tested. Ten of the 21 genotyped fibroblast cultures (see Table 3) were heterozygous for at least 1 SNP site. The results showed that the donor of GM04022 fibroblasts is heterozygous at SNP position rs363125 (NCBI), harboring both an A-allele (adenine) and a C-allele (cytosine) at nucleotide position 5310 in the HD reference sequence NM_002111.6. The fibroblasts from this donor (here called donor-1) were selected for further study. These fibroblasts are from a female Caucasian donor who was 28 years old at the time of cell collection. Her mother, who was 58 years old at the time of the cell collection, was reportedly diagnosed with HD at age 49.

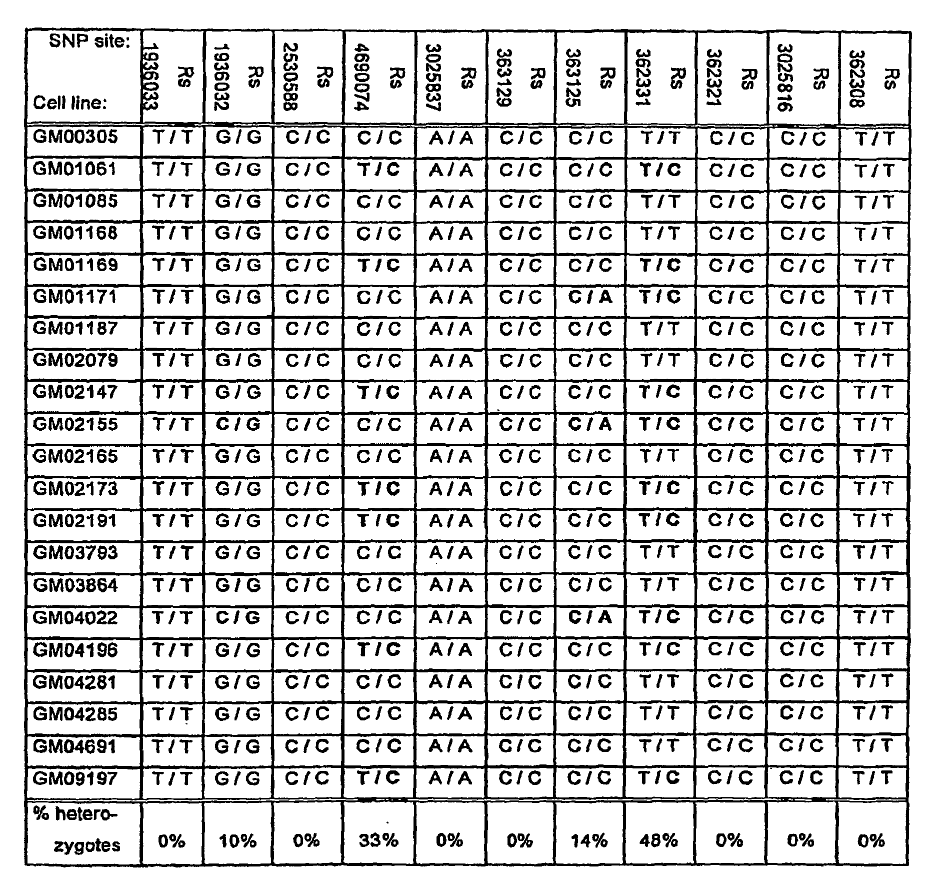

### Development of allele-specific reverse transcription.

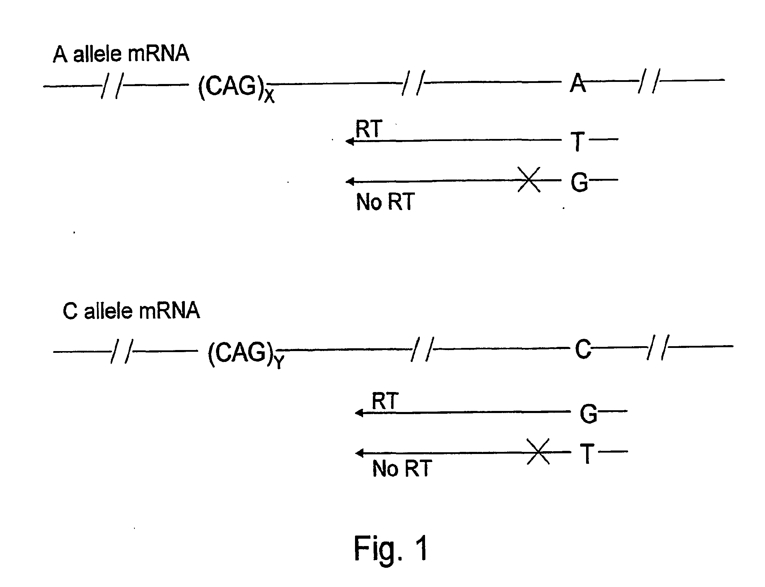

As described above, donot-1 was determined to be heterozygous (adenine versus cytosine) at SNP site rs363125. In order to design an allele-specific RNA interference-based therapy for donor-1, it needs to be determined which of these SNP sequences is associated with the expanded CAG repeat mutation that is located approximately 5000 nucleotides upstream from the SNP position. Appropriate siRNA or shRNA targeting SNP site rs363125 should only reduce protein expression from the allele that contains the expanded CAG repeat and should therefore only be specific for the associated SNP. Correspondence between the SNP identity and the expanded allele cannot be readily determined by cDNA sequencing or by comparing the lengths of PCR products spanning the SNP position and the CAG repeat region. To solve this problem, the inventors developed a strategy that uses SNP-specific reverse transcription (RT) primers to selectively generate cDNA from only one allelic species of HD mRNA. The primers each contained either guanine or thymine at the 3' terminal position, corresponding to SNP site rs363125, as shown in FIG. 1. Primers of various lengths were tested. The 3'-guanine and 3'-thymine primer were each designed to specifically facilitate reverse transcription (also known as first strand synthesis) of only the mRNA containing cytosine or adenine at the SNP position, respectively. Following reverse transcription with either type of RT primer, PCR was conducted on the resulting cDNA using PCR primers that amplify a product spanning the CAG-repeat sequence in the first exon of the HD gene.

RNA isolated from the fibroblasts was reverse transcribed using the Superscript III RT kit (Invitrogen) in the presence of 100 nM of one of the following DNA primers: the 20-mer 5'-GTGTTCTTCTAGCGTTGAA**T**-3', SEQ ID NO: 52 (or a shorter, corresponding 15-mer or 10-mer ending in **T**-3', corresponding to SEQ ID NO: 47 and SEQ ID NO: 42, respectively) or the 20-mer primer 5'-GTGTTCTTCTAGCGTTGAAG-3' SEQ ID NO: 64 (or a shorter, corresponding 15-mer or 10-mer primer ending in **G**-3', corresponding to SEQ ID NO: 59 and SEQ ID NO: 54, respectively) at 100 nM. The CAG repeat sequence on either RT product was then amplified by PCR (Bio-Rad iCycler) using Accuprime GC-Rich DNA-polymerase (Invitrogen), and forward primer 5'-GCCTTCGAGTCCCTCAAGT -3' and reverse primer 5'-GACAATGATTCACACGGTCT-3' at 0.2 µM each (SEQ ID NO: 85 and SEQ ID NO: 86, respectively). The resulting PCR products contain the complete CAG repeat sequence of one of the two alleles of the GM04022 cells. CAG-repeat size for each allelic RT products was determined using standard 1.5% agarose gel electrophoresis with ethidium bromide staining, and also by sequencing of the products of the PCR amplification of the CAG repeat region.

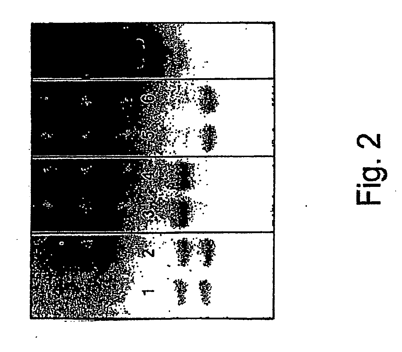

Gel electrophoresis of the respective PCR products (FIG. 2) showed that the RT-PCR assay using the 3'-thymine primer in the RT step yielded a product of approximately 300 base pairs. In contrast, the RT-PCR assay using the 3'-guanine primer in the RT step yielded a PCR product of approximately 360 base pairs. RT primers that were 15 bases in length yielded cDNA from which a PCR product corresponding to only one allele from heterozygous donor-1 was preferentially amplified. Allele specific RT primers of different lengths were also designed and tested for SNP1936032 and SNP362331, both of which were also determined to be heterozygous in donor-1 (Table 3). These experiments showed that primers of 15 bases in length were also optimal for allele-specific transcription from the respective SNP sites.

### Corxespondence between a SNP identity and an individual's expanded allele.

Based on the relative sizes of the PCR products in Figure 2, it may be determined that the CAG-repeat region on the allele containing cytosine at SNP rs363125 contains approximately 20 more repeats than the allele containing adenine at that SNP, with a total CAG-repeat length of about 40 repeats. Sequencing of the PCR products confirmed that the CAG repeat region of the donor-1 allele containing cytosine at SNP position rs363125 contains 44 versus 19 CAG codons, respectively. Therefore, the allele containing cytosine at SNP position rs363125 is the disease-causing allele, and an appropriate allele-specific therapy for donor-1 would deliver an siRNA or shRNA providing the sequence specified here as siRNA 363125_C-16.

### Example 2

### Development of SNP-specific real time PCR assays.

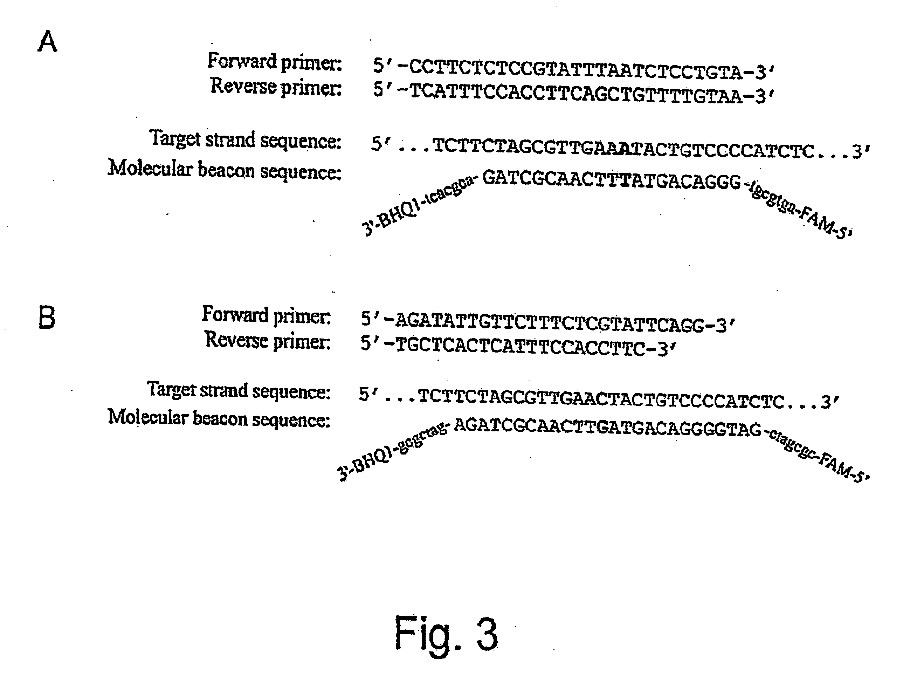

In order to determine whether allele-specific suppression of HD mRNA is occurring in cells, it is necessary to be able to quantify the amount of HD mRNA corresponding to each allele individually. Molecular beacons are synthetic oligonucleotide probes that have a fluorophore and a quencher covalently linked to the respective ends of the oligo. In solution, the beacon adopts a hairpin conformation, causing the fluorophore to be quenched. However, upon hybridization with complementary DNA in a PCR reaction, the hairpin conformation is lost and fluorescence from the fluorophore can be detected. Beacons can be constructed such that as little as a single nucleotide mismatch between the beacon and the complementary DNA is sufficient for the probe to be more stable in its self-annealed state than in the probe-cDNA hybrid. The inventors designed two such beacons corresponding to the two allelic variants of SNP rs363125 (Fig. 3). Absence of genomic DNA carry over in the RNA isolates was confirmed by PCR using forward primer 5'-CTCGCCTCCAGCATGAAAGT-3' and reverse primer 5'-GTGACTGGGGCATTGATT-3', (SEQ ID NOs: 87 and 88, respectively) which amplify monocyte chemo-attractant protein-1 (MCP-1) genomic DNA. Genomic DNA from FieLa cells served as a positive control for the PCR reaction, and only total RNA samples from fibroblasts verified to be without detectable genomic DNA were used in further analyses. Concentration and integrity of the isolated RNA were checked by Experion automated electrophoresis (Bio-Rad, StdSens analysis kit) of the 28S/18S ribosomal RNA. RNA was reverse transcribed into cDNA using the iscript cDNA synthesis kit (BioRad), after which allele-specific real time PCR was conducted using the molecular beacon method. The sequences of the molecular beacons are described in Figure 3 and correspond to SEQ ID NOs: 89 (3'-BHQI-tcacgcaGATCGCAACTT**A**ATGACAGGGtgcgtga-FAM-5') and 90 (3'-BNQI-gcgctagAGATCGCAACTT**C**ATGACAGGGGTAGctagcgc- FAM-5'), for the "A" and the "C" alleles, respectively. In these sequences, the "loop sequence" which binds to a portion of the targeted portion of the PCR product is in capital letters, while the "stem" portions, which form the double strand of the beacon if the beacon is not bound to the targeted portion of the PCR product. The sequences of the primers for the "A" allele correspond to SEQ ID NOs: 91 (5' - CCTTCTCTCCGTATTTAATCTCCTGTA-3') and 92 (5' - TCATTTCCACCTTCAGCTGTTTTGTAA-3') for the forward and the reverse primers, respectively. The sequences of the primers for the "C" allele correspond to SEQ ID NOs 93 (5' - AGATATTGTTCTTTCTCGTATTCAGG-3') and 94 (5' - TGCTCACTCATTTCCACCTTC-3') for the forward and the reverse primers, respectively. Detection of the PCR product was conducted using molecular beacons (Proligo, France) each of which detects only a PCR product containing a complementary nucleotide at the SNP position. The two alleles were quantified in separate PCR reactions, each containing 0.3 µM of the appropriate molecular beacon. During elongation, increase of the FAM fluorescence signal was recorded. The data were quantified against a standard curve generated using a serial dilution of a plasmid containing the PCR product (Baseclear genesynthesis, The Netherlands). Results were normalized against GAPDH, amplified using IQ SYBR Green Supermix (Bio-Rad) with forward primer 5'-ACTCCTCCACCTTTGACGC-3' (SEQ ID NO: 95) and reverse primer 5'-GTTGCTGTAGCCAAATTCGTT-3' (SEQ ID NO: 96).

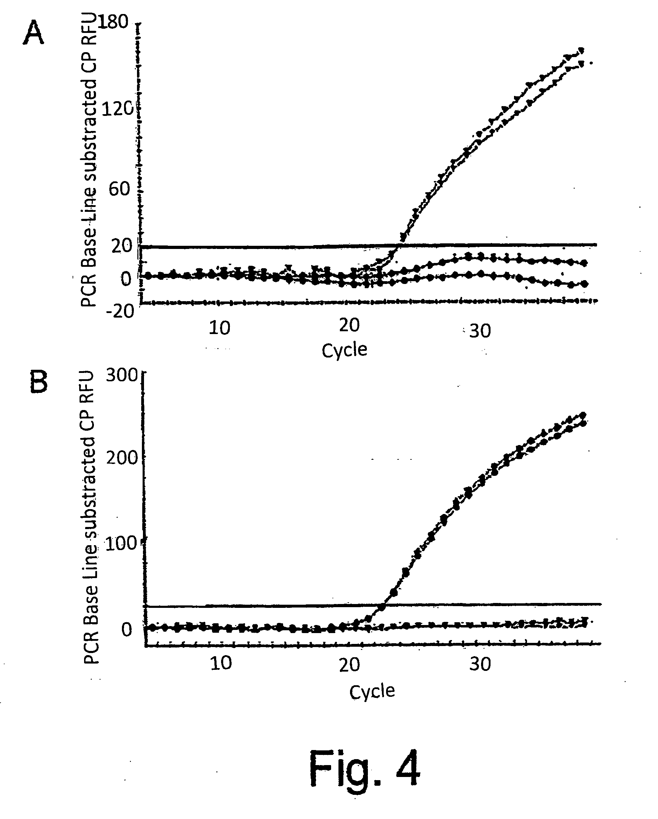

As shown in Figure 4, these two beacons are specific for their respective SNP sequences in real time PCR reactions. In these PCR reactions plasmids were used as template DNA that were engineered and verified to contain one or the other allelic variant of the target sequence. Both beacon MB363125_A and MB363125_C were found to yield a PCR amplification signal only from their corresponding DNA template.

### Allele-specific suppression of huntingtin mRNA.

Fibroblasts were cultured in 25cm² culture flasks (Nunc) as described, but without the addition of PSN antibiotics and Fungizone. Lipofectamine 2000 (Invitrogen) was used to conduct siRNA transfection at three different conditions: 1) mock transfection (n=8); 2) transfection with scrambled siRNA (Ambion) (n=4); and 3) transfection with siRNA sequence 5'-GAAGUACUGUCCCCAUCUCdTdT-3', SEQ ID NO: 97, (Ambion) (n=7) at a concentration of 100 nM. The latter siRNA has a guanine located at position 16 relative to the 3' end of the complementary region of the target huntingtin mRNA, providing specificity for the allele containing cytosine at the SNP position. A parallel cell culture was transfected with a fluorescently labeled Block-It siRNA (Invitrogen) to verify efficiency of Lipofectamine 2000 transfection. The cells were incubated overnight at 37°C, 5% CO₂ and maximum humidity. The cultures were then washed with PBS and fluorescence microscopy (Leica DM-IRB) was used to confirm transfection in the Block-It transfected cultures, which were considered representative for all transfection conditions. The cells were cultured for another day before RNA isolation as described, approximately 48 hours post-transfection.

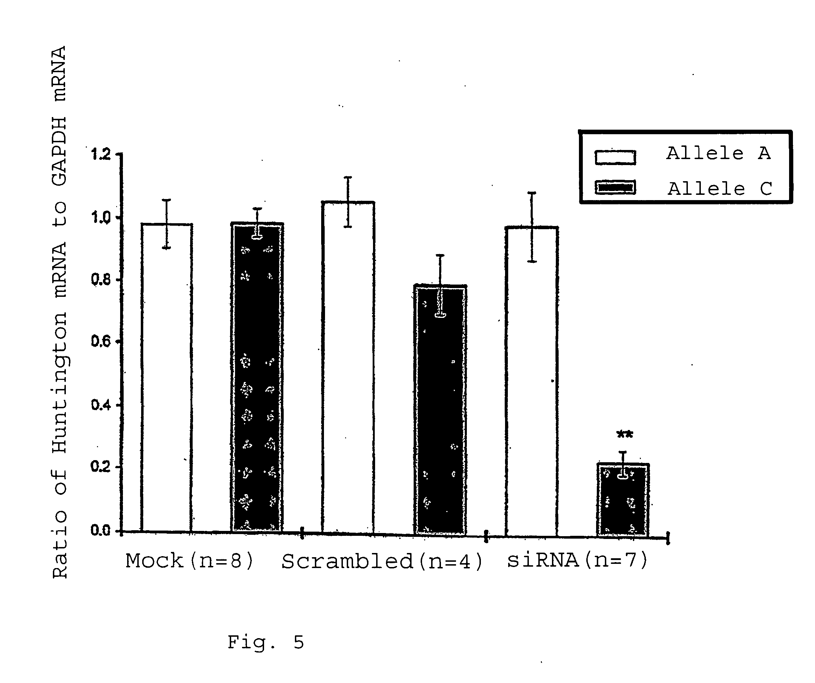

Fibroblasts from donor-1 were transfected with siRNA designed to specifically target the mRNA containing cytosine at SNP site rs363125. This siRNA molecule (siRNA 363125_C-16) was designed such that the cytosine nucleotide of the SNP is located at position 16 relative to the 5' end of the sense strand of the siRNA molecule. The amount of mRNA from both endogenous alleles was separately quantified using the molecular beacons developed to be specific for the allelic variants at this SNP site. The results showed that about 48 hours following treatment of the fibroblasts with siRNA 363125_C-16, mRNA transcripts containing cytosine at position rs363125 were detected at levels approximately 80% lower (p < 0.01, two-tailed) from that detected in controls that were mock transfected, or transfected with a scrambled siRNA (Fig. 5; ANOVA of treatment by allele interaction effect, p = 0.0002). Transcripts containing adenine at position rs363125 were present at levels statistically not different from (p = 0.5145) and numerically similar to the controls.

All publications cited in the specification, both patent publications and non-patent publications, are indicative of the level of skill of those skilled in the art to which this invention pertains. All these publications are herein fully incorporated by reference to the same extent as if each individual publication were specifically and individually indicated as being incorporated by reference.

## Claims

1. A kit for determining which single nucleotide polymorphism variant of an allele from a gene isolated from a heterozygous patient is on the same mRNA transcript as a disease-causing mutation located at a remote region of the gene's mRNA comprising:
a) a first allele-specific primer which recognizes a single nucleotide polymorphism (SNP) variant of a SNP in the mRNA transcript of said heterozygous patient,
b) a second allele-specific primer which recognizes another variant of said SNP in the mRNA transcript of said heterozygous patient, and
c) a set of PCR primers flanking said disease-causing mutation and capable of hybridizing to the remote region of an allele-specific cDNA product,
wherein said allele-specific cDNA product is obtainable from an allele-specific reverse transcription reaction conducted using the allele-specific primer of part (a) or the allele-specific primer of part (b) (but not both) and the mRNA transcript;
said disease-causing mutation is a CAG expansion; and
said set of PCR primers flanking said disease-causing region of part (c) are suitable for amplifying at least a portion of the remote region of said allele specific cDNA.

2. The kit of claim 1, wherein the 3' end of the first or second allele-specific primer is positioned at the single nucleotide polymorphism nucleotide position.

3. The kit of any one of claims 1 and 2, further comprising a third allele-specific primer which recognizes a single variant of a second SNP, and a fourth allele-specific primer which recognizes another variant of said second SNP, wherein said second SNP is different from the SNP recognized by the first and the second allele-specific primers, and
wherein the 3' end of the first, the second, the third, or the fourth allele-specific primer is positioned at the single nucleotide polymorphism nucleotide position and wherein the disease is Huntington's disease, and both of said SNP recognized by the first and the second allele-specific primers and said second single nucleotide polymorphism are selected from rs1936032, rs4690074, rs363125, and rs362331.

4. The kit of any one of claims 2 to 3, wherein said disease is Huntington's disease and the 3' end of the first allele-specific primer is selected from the group consisting of SEQ ID NO:41 - SEQ ID NO:64.

5. The kit of claim 4, wherein the third allele-specific primer is selected from the group consisting of SEQ ID NO:53 - SEQ ID NO:64 and the first allele-specific primer is selected from the group consisting of SEQ ID NO:41 - SEQ ID NO:52.

6. The kit of claim 5, wherein the first allele-specific primer is SEQ ID NO:47 or SEQ ID NO:59.

7. The kit of any one of claims 1 and 2, wherein the disease is Huntington's disease, and the single nucleotide polymorphism is selected from rs1936032, rs4690074, rs363125, and rs362331.

8. The kit of claim 3, wherein said second single nucleotide polymorphism is different from the single nucleotide polymorphism recognized by the first and the second allele-specific primers.

9. The kit of claim 8, further comprising at least:
f) a fifth allele-specific primer which recognizes one variant of a third single nucleotide polymorphism, and
g) a sixth allele-specific primer which recognizes another variant of said third single nucleotide polymorphism, wherein
the SNP recognized by the first and the second allele-specific primers, the second single nucleotide polymorphism, and said third single nucleotide polymorphism are different, wherein further said SNP recognized by the first and the second allele-specific primers, the second single nucleotide polymorphism, and said third single nucleotide polymorphism are selected from rs1936032, rs4690074, rs363125, and rs362331.

10. The kit of claim 9, further comprising:
h) at least a seventh allele-specific primer which recognizes one variant of a fourth single nucleotide polymorphism, and
i) at least an eighth allele-specific primer which recognizes another variant of said fourth single nucleotide polymorphism, wherein said SNP recognized by the first and the second allele-specific primers, said second single nucleotide polymorphism, said third single nucleotide polymorphism and said fourth single nucleotide polymorphism are different and are selected from rs1936032, rs4690074, rs363125, and rs362331.

11. The kit of claim 1, wherein the 3' end of the allele-specific primer is positioned at the single nucleotide polymorphism nucleotide position.

12. The kit of claim 1, wherein the 3' end of the allele-specific primer is not positioned at the single nucleotide polymorphism nucleotide position.


SEQUENCE LISTING

<110>  Paul van Bilsen, William F. Kaemmerer and Eric Burright

<120>  METHODS AND KITS FOR LINKING POLYMORPHIC SEQUENCES TO EXPANDED
       REPEAT MUTATIONS

<130>  P0024231.02

<140>  PCT/US2007/12259
<141>  2007-05-23

<150>  11/439,858
<151>  2006-05-24

<160>  97

<170>  PatentIn version 3.4

<210>  1
<211>  9
<212>  DNA
<213>  Homo sapiens

<400>  1
aagcctaag                                                              9


<210>  2
<211>  10
<212>  DNA
<213>  Homo sapiens

<400>  2
taagcctaag                                                             10


<210>  3
<211>  11
<212>  DNA
<213>  Homo sapiens

<400>  3
gtaagcctaa g                                                           11


<210>  4
<211>  12
<212>  DNA
<213>  Homo sapiens

<400>  4
agtaagccta ag                                                          12


<210>  5
<211>  13
<212>  DNA
<213>  Homo sapiens

<400>  5
gagtaagcct aag                                                         13


<210>  6
<211>  14
<212>  DNA
<213>  Homo sapiens

<400>  6
cgagtaagcc taag                                                        14

<210> 7
<211> 15
<212> DNA
<213> Homo sapiens

<400> 7
acgagtaagc ctaag 15

<210> 8
<211> 16
<212> DNA
<213> Homo sapiens

<400> 8
aacgagtaag cctaag 16

<210> 9
<211> 17
<212> DNA
<213> Homo sapiens

<400> 9
gaacgagtaa gcctaag 17

<210> 10
<211> 18
<212> DNA
<213> Homo sapiens

<400> 10
ggaacgagta agcctaag 18

<210> 11
<211> 9
<212> DNA
<213> Homo sapiens

<400> 11
aagcctaac 9

<210> 12
<211> 10
<212> DNA
<213> Homo sapiens

<400> 12
taagcctaac 10

<210> 13
<211> 11
<212> DNA
<213> Homo sapiens

<400> 13
gtaagcctaa c 11

<210> 14
<211> 12
<212> DNA
<213> Homo sapiens

<400> 14

agtaagccta ac                                                          12

<210>  15
<211>  13
<212>  DNA
<213>  Homo sapiens

<400>  15
gagtaagcct aac                                                         13

<210>  16
<211>  14
<212>  DNA
<213>  Homo sapiens

<400>  16
cgagtaagcc taac                                                        14

<210>  17
<211>  15
<212>  DNA
<213>  Homo sapiens

<400>  17
acgagtaagc ctaac                                                       15

<210>  18
<211>  16
<212>  DNA
<213>  Homo sapiens

<400>  18
aacgagtaag cctaac                                                      16

<210>  19
<211>  17
<212>  DNA
<213>  Homo sapiens

<400>  19
gaacgagtaa gcctaac                                                     17

<210>  20
<211>  18
<212>  DNA
<213>  Homo sapiens

<400>  20
ggaacgagta agcctaac                                                    18

<210>  21
<211>  9
<212>  DNA
<213>  Homo sapiens

<400>  21
gctgagcgg                                                               9

<210>  22
<211>  10
<212>  DNA
<213>  Homo sapiens

<400>  22
ggctgagcgg                                                              10

<210>  23
<211>  11
<212>  DNA
<213>  Homo sapiens

<400>  23
aggctgagcg g                                                            11

<210>  24
<211>  12
<212>  DNA
<213>  Homo sapiens

<400>  24
aaggctgagc gg                                                           12

<210>  25
<211>  13
<212>  DNA
<213>  Homo sapiens

<400>  25
caaggctgag cgg                                                          13

<210>  26
<211>  14
<212>  DNA
<213>  Homo sapiens

<400>  26
ccaaggctga gcgg                                                         14

<210>  27
<211>  15
<212>  DNA
<213>  Homo sapiens

<400>  27
tccaaggctg agcgg                                                        15

<210>  28
<211>  16
<212>  DNA
<213>  Homo sapiens

<400>  28
atccaaggct gagcgg                                                       16

<210>  29
<211>  17
<212>  DNA
<213>  Homo sapiens

<400>  29
catccaaggc tgagcgg                                                      17

<210>  30
<211>  18
<212>  DNA
<213>  Homo sapiens

<400>  30
acatccaagg ctgagcgg                                                    18


<210>  31
<211>  9
<212>  DNA
<213>  Homo sapiens

<400>  31
gctgagcga                                                               9


<210>  32
<211>  10
<212>  DNA
<213>  Homo sapiens

<400>  32
ggctgagcga                                                             10


<210>  33
<211>  11
<212>  DNA
<213>  Homo sapiens

<400>  33
aggctgagcg a                                                           11


<210>  34
<211>  12
<212>  DNA
<213>  Homo sapiens

<400>  34
aaggctgagc ga                                                          12


<210>  35
<211>  13
<212>  DNA
<213>  Homo sapiens

<400>  35
caaggctgag cga                                                         13


<210>  36
<211>  14
<212>  DNA
<213>  Homo sapiens

<400>  36
ccaaggctga gcga                                                        14


<210>  37
<211>  15
<212>  DNA
<213>  Homo sapiens

<400>  37
tccaaggctg agcga                                                       15


<210>  38
<211>  16
<212>  DNA

<213>  Homo sapiens

<400>  38
atccaaggct gagcga                                                        16


<210>  39
<211>  17
<212>  DNA
<213>  Homo sapiens

<400>  39
catccaaggc tgagcga                                                       17


<210>  40
<211>  18
<212>  DNA
<213>  Homo sapiens

<400>  40
acatccaagg ctgagcga                                                      18


<210>  41
<211>  9
<212>  DNA
<213>  Homo sapiens

<400>  41
gcgttgaat                                                                 9


<210>  42
<211>  10
<212>  DNA
<213>  Homo sapiens

<400>  42
agcgttgaat                                                               10


<210>  43
<211>  11
<212>  DNA
<213>  Homo sapiens

<400>  43
tagcgttgaa t                                                             11


<210>  44
<211>  12
<212>  DNA
<213>  Homo sapiens

<400>  44
ctagcgttga at                                                            12


<210>  45
<211>  13
<212>  DNA
<213>  Homo sapiens

<400>  45
tctagcgttg aat                                                           13


<210>  46
<211>  14

<212>  DNA
<213>  Homo sapiens

<400>  46
ttctagcgtt gaat                                                          14


<210>  47
<211>  15
<212>  DNA
<213>  Homo sapiens

<400>  47
cttctagcgt tgaat                                                         15


<210>  48
<211>  16
<212>  DNA
<213>  Homo sapiens

<400>  48
tcttctagcg ttgaat                                                        16


<210>  49
<211>  17
<212>  DNA
<213>  Homo sapiens

<400>  49
ttcttctagc gttgaat                                                       17


<210>  50
<211>  18
<212>  DNA
<213>  Homo sapiens

<400>  50
gttcttctag cgttgaat                                                      18


<210>  51
<211>  19
<212>  DNA
<213>  Homo sapiens

<400>  51
tgttcttcta gcgttgaat                                                     19


<210>  52
<211>  20
<212>  DNA
<213>  Homo sapiens

<400>  52
gtgttcttct agcgttgaat                                                    20


<210>  53
<211>  9
<212>  DNA
<213>  Homo sapiens

<400>  53
gcgttgaag                                                                 9


<210>  54

<211>  10
<212>  DNA
<213>  Homo sapiens

<400>  54
agcgttgaag                                                              10


<210>  55
<211>  11
<212>  DNA
<213>  Homo sapiens

<400>  55
tagcgttgaa g                                                            11


<210>  56
<211>  12
<212>  DNA
<213>  Homo sapiens

<400>  56
ctagcgttga ag                                                           12


<210>  57
<211>  13
<212>  DNA
<213>  Homo sapiens

<400>  57
tctagcgttg aag                                                          13


<210>  58
<211>  14
<212>  DNA
<213>  Homo sapiens

<400>  58
ttctagcgtt gaag                                                         14


<210>  59
<211>  15
<212>  DNA
<213>  Homo sapiens

<400>  59
cttctagcgt tgaag                                                        15


<210>  60
<211>  16
<212>  DNA
<213>  Homo sapiens

<400>  60
tcttctagcg ttgaag                                                       16


<210>  61
<211>  17
<212>  DNA
<213>  Homo sapiens

<400>  61
ttcttctagc gttgaag                                                      17

<210> 62
<211> 18
<212> DNA
<213> Homo sapiens

<400> 62
gttcttctag cgttgaag 18


<210> 63
<211> 19
<212> DNA
<213> Homo sapiens

<400> 63
tgttcttcta gcgttgaag 19


<210> 64
<211> 20
<212> DNA
<213> Homo sapiens

<400> 64
gtgttcttct agcgttgaag 20


<210> 65
<211> 9
<212> DNA
<213> Homo sapiens

<400> 65
cacacagtg 9


<210> 66
<211> 10
<212> DNA
<213> Homo sapiens

<400> 66
gcacacagtg 10


<210> 67
<211> 11
<212> DNA
<213> Homo sapiens

<400> 67
tgcacacagt g 11


<210> 68
<211> 12
<212> DNA
<213> Homo sapiens

<400> 68
gtgcacacag tg 12


<210> 69
<211> 13
<212> DNA
<213> Homo sapiens

<400> 69
agtgcacaca gtg 13

<210> 70
<211> 14
<212> DNA
<213> Homo sapiens

<400> 70
aagtgcacac agtg 14


<210> 71
<211> 15
<212> DNA
<213> Homo sapiens

<400> 71
gaagtgcaca cagtg 15


<210> 72
<211> 16
<212> DNA
<213> Homo sapiens

<400> 72
tgaagtgcac acagtg 16


<210> 73
<211> 17
<212> DNA
<213> Homo sapiens

<400> 73
atgaagtgca cacagtg 17


<210> 74
<211> 18
<212> DNA
<213> Homo sapiens

<400> 74
gatgaagtgc acacagtg 18


<210> 75
<211> 9
<212> DNA
<213> Homo sapiens

<400> 75
cacacagta 9


<210> 76
<211> 10
<212> DNA
<213> Homo sapiens

<400> 76
gcacacagta 10


<210> 77
<211> 11
<212> DNA
<213> Homo sapiens

<400> 77
tgcacacagt a 11

<210>  78
<211>  12
<212>  DNA
<213>  Homo sapiens

<400>  78
gtgcacacag ta                                                          12


<210>  79
<211>  13
<212>  DNA
<213>  Homo sapiens

<400>  79
agtgcacaca gta                                                         13


<210>  80
<211>  14
<212>  DNA
<213>  Homo sapiens

<400>  80
aagtgcacac agta                                                        14


<210>  81
<211>  15
<212>  DNA
<213>  Homo sapiens

<400>  81
gaagtgcaca cagta                                                       15


<210>  82
<211>  16
<212>  DNA
<213>  Homo sapiens

<400>  82
tgaagtgcac acagta                                                      16


<210>  83
<211>  17
<212>  DNA
<213>  Homo sapiens

<400>  83
atgaagtgca cacagta                                                     17


<210>  84
<211>  18
<212>  DNA
<213>  Homo sapiens

<400>  84
gatgaagtgc acacagta                                                    18


<210>  85
<211>  19
<212>  DNA
<213>  Homo sapiens

<400>  85

gccttcgagt ccctcaagt                                                    19

<210>  86
<211>  20
<212>  DNA
<213>  Homo sapiens

<400>  86
gacaatgatt cacacggtct                                                   20

<210>  87
<211>  20
<212>  DNA
<213>  Homo sapiens

<400>  87
ctcgcctcca gcatgaaagt                                                   20

<210>  88
<211>  18
<212>  DNA
<213>  Homo sapiens

<400>  88
gtgactgggg cattgatt                                                     18

<210>  89
<211>  35
<212>  DNA
<213>  Homo sapiens

<400>  89
tcacgcagat cgcaacttaa tgacagggtg cgtga                                  35

<210>  90
<211>  40
<212>  DNA
<213>  Homo sapiens

<400>  90
gcgctagaga tcgcaacttc atgacagggg tagctagcgc                             40

<210>  91
<211>  27
<212>  DNA
<213>  Homo sapiens

<400>  91
ccttctctcc gtatttaatc tcctgta                                           27

<210>  92
<211>  27
<212>  DNA
<213>  Homo sapiens

<400>  92
tcatttccac cttcagctgt tttgtaa                                           27

<210>  93
<211>  26
<212>  DNA
<213>  Homo sapiens

<400>  93
agatattgtt ctttctcgta ttcagg                                          26


<210>  94
<211>  21
<212>  DNA
<213>  Homo sapiens

<400>  94
tgctcactca tttccacctt c                                               21


<210>  95
<211>  19
<212>  DNA
<213>  Homo sapiens

<400>  95
actcctccac ctttgacgc                                                  19


<210>  96
<211>  21
<212>  DNA
<213>  Homo sapiens

<400>  96
gttgctgtag ccaaattcgt t                                               21


<210>  97
<211>  21
<212>  DNA
<213>  Artificial

<220>
<223>  siRNA sequence

<400>  97
gaaguacugu ccccaucuct t                                               21